# EUROPEAN PATENT SPECIFICATION

(11) **EP 3 005 884 B1**
(45) Date of publication and mention of the grant of the patent: **19.04.2017**
(21) Application number: 15189164.5
(22) Date of filing: 09.10.2015
(51) Int. Cl.: A23L 27/10, A23L 7/126, A21D 2/26, A21D 2/36, A23L 2/56, A61Q 11/00, A61K 8/97, A61K 36/232, A23L 23/00

(54) **TASTE MODULATOR AND METHOD OF USE THEREOF**
GESCHMACKSMODULATOR UND VERFAHREN ZUR VERWENDUNG DAVON
MODULATEUR DE GOÛT ET SON PROCÉDÉ D'UTILISATION

(30) Priority: 10.10.2014 US 201414511963
(43) Date of publication of application: 13.04.2016
(73) Proprietor: International Flavors & Fragrances Inc., New York, NY 10019 (US)
(72) Inventor: CHIN, Hsi-Wen, Tinton Falls, NJ 07724 (US); JOHN, Thumpalasseril V., Morganville, NJ 07751 (US); KIM, Jung-A, Edgewater, NJ 07020 (US); SINGH, Ajay Pratap, Highland Park, NJ 08904 (US)
(74) Representative: Lawrence, John

(56) References cited:
- EP-A1- 2 641 479
- WO-A1-2013/085014
- JP-A- 2006 191 826
- JP-A- 2007 176 919
- JP-B1- 4 606 505

## Description

### Background

Health practitioners have been promoting the benefits of whole grain foods. The importance of increasing whole grain consumption is reflected in the changes in recommendations set forth by government and health organization expert groups. In the Healthy People 2010 Report (National Academy Press, 1999), it is recommended that for a 2,000 calorie diet, individuals two years and older should consume at least six daily servings of grain products with at least three being whole grains. In the 2005 Dietary Guidelines for Americans it is recommended that consumers should eat three or more ounce equivalents of whole grain foods each day, with the rest coming from enriched or whole grain flours. In general, at least half of the intake should come from whole grains. The American Heart Association, American Diabetes Association and the American Cancer Society also make specific recommendations regarding increasing the consumption of whole grains.

Despite the well-documented nutritional advantages of eating whole grain products as compared to refined flour products, consumers often like refined breads better than whole wheat breads, indicating that sensory properties are a barrier to consumption of whole wheat bread (Bakke & Vickers (2007) J. Food Sci. 72:S473-S480; Moskowitz, et al. (2012) J. Agric. Food Chem. 60:11245-11252). Currently, in the United States, consumption of white bread made from refined wheat flour is greater than that of whole wheat bread. Such consumer preference for refined flour products may limit the use of current whole grain wheat flours in bakery and snack products.

Improving the palatability of whole grain foods would help promote the acceptance and consumption of the healthful whole grain foods by consumers. In this respect, the use of methylsulfonylmethane to reduce bitterness and undesirable flavors in various food products, including grain products, has been suggested. See WO 2010/141889. WO 2013/085014 and JP 2007176919 describe a taste-masking compositions comprising a vegetable extract, including *Angelica keiskei.* However, there remains a need in the art to develop nutritious, high fiber food products and fiber supplements which have a texture and taste more palatable to a number of consumers.

### Summary of the Invention

The invention provides a method of reducing an undesirable taste in a product selected from the group consisting of a food product, a dental product, an oral hygiene product and a medicinal product comprising the step of incorporating a taste-masking composition, wherein the taste-masking composition comprises a taste-masking effective amount ranging from 1 to 5000 ppm by weight of the composition of an Angelica root extract, and wherein the Angelica root extract is prepared by a process comprising the steps of: (i) submerging a root of an Angelica sinensis in a solvent selected from the group consisting of water, an organic solvent and a mix thereof; (ii) circulating the solvent continuously through the root of the Angelica sinensis at a temperature ranging from room temperature to 100 °C to provide a soluble extract; and (iii) collecting the soluble extract from step (ii) and evaporating the solvent to provide the Angelica root extract.

The present invention relates to a novel taste-masking composition and its unexpected advantageous use in reducing an undesirable taste in a food product (*e.g.,* a beverage, broth, or whole grain food product), a dental product, an oral hygiene product or a medicinal product. Specifically, the present invention relates to the taste-masking composition comprising a taste-masking effective amount of an *Angelica* sinensis. extract.

The present invention also relates to the surprising finding of the unexpected effectiveness of the taste-masking composition provided above in reducing an undesirable taste in a food product (*e.g.,* a beverage, broth, or whole grain food product), a dental product, an oral hygiene product or a medicinal product.

The present invention further relates to a method of reducing an undesirable taste in a food product, a dental product, an oral hygiene product or a medicinal product through the incorporation of the taste-masking composition provided above.

These and other embodiments of the present invention will be apparent by reading the following specification.

### Brief Description of the Drawings

**Figure 1** shows the flavor profile of a wheat cracker containing an extract of *Angelica sinensis* root. N=11. * Directional difference at p=0.10. ** Significant difference between the two samples at p=0.05.
**Figure 2** shows the number of samples preferred in a paired comparison test between a basic wheat cracker and a wheat cracker treated with *Angelica* root extract. N=22.

### Detailed Description of the Invention

An extract of *Angelica* root is known for its medicinal use. It also has the organoleptic characteristics of herbaceous, celery, hay, grainy, woody, bitter, dried and cardboard and is therefore used in perfumery and flavoring. It has now been surprisingly found that at a taste-masking effective amount the *Angelica* sinensis root extract is effective in masking the off-flavors associated with various products and, in particular, food products such as whole grain foods. Specifically, water and/or organic solvent extracts of root samples of *A. sinensis* and *A. archangelica* improve the taste of whole grain foods by reducing bitter and astringent tastes and the harsh mouthfeel of whole grain products such as bread, crackers and pasta. In addition, *Angelica* root extracts were found to reduce bitter, metallic, astringent, and off-taste/linger of beverages and broths. Therefore, the present invention provides a method for reducing an undesirable taste of various products such as food products by adding an *Angelica* sinensis root extract as described herein.

*Angelica sinensis,* also known as Dong Quai, is perennial herb used in traditional Chinese herbal medicine. *A. sinensis* contains a number of compounds including, but not limited to vitamin B12, vitamin E, carotene, pantothenic acid, folic acid, folinic acid, biotin, nicotinic acid, nicotinamide, sucrose, butylidene phthalide, n-valerophenone-o-carboxylic acid dehydrophthalic anhydride, beta-sitosterol, sitosterol-D-glucoside, monoterpene hydrocarbons, monoterpene alcohols, oxygenated sesquiterpenes, ethyl ethers of monoterpene alcohols, ethyl sterols of long chain fatty acids, falcarinol, falcarindiol, falcarinolone, choline, scopoletin, archangelicin, 8(S),9(R)-9-angeloyl-oxy-8,9-dihydrooroselol, psoralen, bergapten, xanthotoxin, chalcones such as asitaba-chalcone, 4-hydroxyderricin, water soluble tannin, α-pinene, myrcene, p-cymene, oxypeucedanin, imperatorin, isoimeratorin, ferulic acid, columbianetin, columbianetin acetate, columbiadin, osthol, isoimperatorin, columbianetin-β-D-glucopyranoside, and AR-4E-2, 1, safrole, isosafrole, n-dodecanol, n-tetradecanol, palmitic acid. See, Monograph Angelica sinensis (2004) Alter. Med. Rev. 9:429-433; Noé (1998) J. Naturopathic Med. 7:66-72. In addition, *A. sinensis* contains alkyl phthalides such as ligustilide, (Z)-ligustilide, (Z)-6,7-epoxyligustilide, angelicide, (Z)-butylidene phthalide, butyl-phthalide, and 2,4-dihydrophthalic anhydride, which are the major components of the essential oil fraction of the roots. The non-volatile constituents reported are phenylpropanoids ((E)-ferulic acid coniferyl ferulate); benzenoids (valerophenone-o-carboxylic acid and vanillic acid); and coumarins (angelol G, angelicone and umbelliferone. See, WHO Monographs on Selected Medicinal Plants (2004) Vol. 2, pages 25-34. Other known components found in the root of *A. sinensis* include, but are not limited to, alanine, α-tocopherol, angelic acid, *Angelica* polymorpha alkaloid, *Angelica* polysaccharide-AS-1, compound E-232, angelicone, angelol, arachidonic acid, brefeldin A, cadinene, carvacrol, choline, dodecan-1-ol, GABA, gamma-aminobutyric acid, lignoceric acid, n-tetradecane, n-valerophenone-o-carbonic acid, and neoangelide. See, Dr. Duke's *Phytochemical and Ethnobotanical Databases* provided by the Agricultural Research Service, Germplasm Resources Information Network.

Like *A. sinensis,* the root of *A. archangelica* (also known as *A. officinalis* or garden *Angelica*) is also known contain one or more of the following compounds: 1,1-diethoxyhexane, 1,1-diethoxyoctane, 12-methyl-13-tridecanolide, 12-methyl-omega-tridecanolide, 13-tridecanolide, 15-hydroxypentadecanoic acid, 15-pentadecanolide, 17-heptadecanolide, 2'-angeloyl-3'-isovaleryl-vaginate, 2-decanone, 2-nonanone, 2-pentyl-furan, 4,4,6-trimethylcyclohex-2-en-1-one, 4-ehtoxy-1-P-menthene, 4-methoxy-7H-furo(3,2-G)(1)benzopyran-7-one, 5-undecen-3-yne, 7-methoxy-8-(3-methyl-2-butenyl)-2H-1-benzopyran-2-one, 8-(2-(3-methylbutoxy)-3-hydroxy-3-methylbutoxy)psoralen, adenosine, α-caryophyllene, α-copaen-11-ol, α-copaen-8-ol, α-copaene, α-cubene, α-humulene, α-phellandrene, α-phellandrene-8-ol, α-pinene, α-terpinene, α-terpineol, α-terpinolene, α-thujene, *Angelicain*, angelicin, apterin, AR-curcumene, archangelenone, archangelicin, archangelin, bergapten, β-bisabolene, β-cadrene, β-copaene, β-elemene, β-eudesmol, β-farnesene, β-himachalene, β-myrcene, β-phellandrene, β-pinene, β-selinene, β-sesquiphellandrene, β-thujene, bicyclogermacrene, borneol, borneol-acetate, bornyl-acetate, bornyl-isovalerate, byakangelicin, byakangelicin-2'-o-isovalerate, byakangelicin-angelate, byakangelicol, camphene, car-3-ene, carvone, caryophyllene, cedrol, chrysanthenol-acetate, chrysanthenol-acetate-I, chrysanthenol-acetate-II, cis-1-ethoxy-2-P-menthene, cis-3-ethoxy-1-p-menthene, cis-4-ethoxy-2-pinene, cis-4-ethoxy-thujane, cis-β-farnesene, cis-carvyl-acetate, cis-ocimene, cis-p-menth-2-en-1-ol, cis-piperitol, cis-sabinene-hydrate, cis-thujenol, cis-verbenol, coniferin, cryptone, cuminyl-alcohol, cuparene, cymen-8-ol, cymene, D-α-phellandrene, deca-trans-2,trans-4-dien-1-al, delta-3-carene, delta-cadinene, delta-elemene, diacetyl, dihydroeudesmol, diprenyl-naringenin, eicosane, elemol, ethyl-caprate, ethyl-laurate, ethyl-linoleate, ethyl-linolenate, ethyl-myristate, ethyl-oleate, ethyl-palmitate, ethyl-pentadecanoate, ethyl-stearate, furfural, gamma-cadinene, gamma-caryophyllene, gamma-muurolene, gamma-terpinene, gamma, terpineol, germacrene-D, germanium, heptadecan-1-ol-acetate, heptadecane, heptyl-isovalerate, heraclenol-2'-o-isovalerate, heraclenol-2'-o-senecioate, hexadecan-1-ol, hexadecane, humulene-monoxide, imperatorin, isoamyl-isovalerate, isoimperatorin, isopimpinellin, ligustilide, limonene, linalool, m-cymen-8-ol, malic acid, malonic acid, methyl-ethyl-acetic acid, myrcene, nonadecane, nonanal, octadecan-1-ol, octadecane, octanal, octyl-isovalerate, osthenol, osthenole, osthol, ostruthol, oxypeucedanin, oxypeucedanin-hydrate, oxypeucidanin, oxypeucidanin hydrate, oxypeucidanin methanolate, p-cymen-8-ol, p-cymene, pentadecan-1-al, pentadecanal, pentadecanolide, peucenin-7-methylether, phellandral, phellopterin, piperitone, prangolarin, psoralen, rosifoliol, sabinene, sabinol, sabinyl acetate, scopoletin, spathulenol, tannins, terpinen-4-ol, terpinolene, tetradecan-1-al, tetradecan-1-ol acetate, tetradecanal, tetradecane, thujopsene, trans-1-ethoxy-2-p-menthene, trans-3-ethoxy-1-p-menthane, trans-4-ethoxy-2-pinene, trans-4-ethoxy-thujane, trans-anethole, trans-beta-farnesene, trans-carveol, trans-carvyl acetate, trans-ocimene, trans-p-menth-2-en-1-ol, trans-pinocarvyl acetate, trans-piperitol, trans-sabinene hydrate, verbenol, trans-verbenyl-2-methyl butyrate, trans-verbenyl acetate, trans-verbenyl isovalerate, tridecan-1-ol, tridecanolide, umbelliferone, umbelliferose, umbelliprenin, undec-5-en-3-yne, xanthotoxin, zingiberene. See Duke (1992) Handbook of phytochemical constituents of GRAS herbs and other economic plants. Boca Raton, FL. CRC Press; Härmälä, et al. (1992) Phytochem. Anal. 3(1):42-48; and Dr. Duke's *Phytochemical and Ethnobotanical Databases supra.*

In addition to *A. sinensis* and *A. archangelica,* the present invention also includes the use of extracts from the root of other species of *Angelica* including *e.g., A. acutiloba, A. atropurpurea* (American *Angelica* or purple *Angelica*)*, A. crucifolia, A. dahurica* (Bai Zhi), *A. decursiva, A. edulis, A. genuflexa* (Kneeling *Angelica*)*, A. gigas* (Giant *Angelica*)*, A. glauca, A. japonica, A. keiskei, A. koreana, A. megaphylla, A. Montana, A. polymorpha, A. pubescens* (Du Huo), *A. silvestris* (Wild *Angelica*)*,* and/or *A. ursine*, which are known to be edible. See Plants for a Future database available on the world-wide web.

An extract of the present invention is prepared by contacting a root sample of an *Angelica* sinensis with water, an organic solvent, or a combination thereof, to extract compounds from the root sample. In one embodiment, the root sample is afresh. In another embodiment, the root sample is dry. In a further embodiment, the root sample is pulverized, crushed, milled, pulped, minced, ground and/or powdered prior to extraction. In one embodiment, the extract is prepared with water. In another embodiment, an organic solvent is used. In another embodiment, the organic solvent is miscible with water. An example of suitable organic solvents includes, but is not limited to an alcohol such as ethanol, acetone, methanol, n-propanol or iso-propanol. In a particular embodiment, the organic solvent is ethanol, *e.g*., 200 proof (absolute ethanol), 190 proof (95% ethanol) or 180 proof (90% ethanol). In yet another embodiment, a combination of water and one or more of the above-referenced organic solvents is used to prepare the *Angelica* root extract. The ratio of water to organic solvent can vary and is desirably in the range of 10-90% water to 10-90% organic solvent. More preferably, the ratio of water to organic solvent is 70:30, 60:40, 50:50, 40:60 or 30:70. The solvent is circulated continuously through the *Angelica* root at a temperature between room temperature and 100 °C, preferably at a temperature higher than room temperature but lower than 100 °C and more preferably between 70 and 80 °C for a period of time that is sufficient to solubilize a taste-masking agent. In one embodiment, the circulation time is between 2 and 6 hours and preferably between 3 and 5 hours. The resulting liquid extract is subsequently separated from the insoluble plant material. These steps can be repeated multiple times by adding fresh solvent to the insoluble plant material. The liquid extracts are pooled and solvent is evaporated to produce a dry powdered extract of *Angelica* root. Once the soluble compounds from the root sample are extracted, the insoluble and particulate material is removed, *e.g*., by centrifugation, sedimentation, or filtration.

The process for preparing an Angelica sinensis root extract of the present invention comprises the steps of:
(i) submerging a root of an *Angelica* sinensis plant in a solvent selected from the group consisting of water, an organic solvent and a mix thereof;
(ii) circulating the solvent continuously through the root of the *Angelica* plant at a temperature ranging from room temperature to 100 °C to provide a soluble extract; and
(iii) collecting the soluble extract from step (ii) and evaporating the solvent to provide the *Angelica* root extract.

The extraction processed according to the disclosed processes provides the *Angelica* sinensis root extract of the present invention, which is a mixture of *Angelica root* components that are soluble in water and/or the organic solvents, with a yield of about 60% by weight or less.

The extract can be used as is in the methods described herein or further processed without affecting the desired functions. An example of further processing includes washing the extract with an organic solvent to remove intrinsic flavors of *Angelica* root. The organic solvent used to wash the extract is desirably not water miscible and can include, but is not limited to, n-butanol, chloroform, 1,2-dichloroethane, ethyl acetate, or diethyl ether. In particular embodiments, the organic solvent is 2-butanol.

The extract may also be further fractionated to a single compound or combination of compounds that have the desired activity of improving the taste of whole grain foods. Such fractionation can be carried out by well-known methods including, but not limited to, precipitation, centrifugation, filtration, ultrafiltration, selective digestion, extraction, chromatography, electrophoresis or complex formation. Each resulting subfraction may be assayed for the desired activity using the original assay until a pure, active agent is obtained.

As indicated, water and/or organic solvent extraction of the root of Angelica sinensis results in an extract having the function of improving the taste of various food products, in particular beverages, broths and whole grain food products. Accordingly, the present invention provides an extract of an *Angelica* root and a method for using the same to improve the taste of food products. In some embodiments, the extract includes, but is not limited to, one or more of the above-referenced components found in species of the genus *Angelica.* In accordance with the method of the invention, the *A. sinensis* root extract is added to a food product before, during or after preparation. In this respect, the *A. sinensis* root extract can be a component of the food product (*i.e.,* a food additive), or a spread, condiment, sauce, coating, glaze, or topping applied to the food product thereby improving the taste of the food product. In this respect, the extract can be provided as a liquid, semi-liquid, solid, semi-solid, powder, granule, etc. By improving the taste, it is meant that the *Angelica* root extract has been demonstrated to reduce the bitter, metallic, and astringent off-taste/linger of food products, as well as reduce the harsh mouthfeel, *e.g*., grainy-rough texture cardboardy, chalky, and/or mouth-drying attributes, of whole grain products.

As used herein, "a taste-masking agent" is understood to mean a compound that, when used in a product including a food product, a dental product, an oral hygiene product or a medicinal product, provides perceived reduction of an undesirable taste (*e.g*., bitterness, astringency, off taste of an artificial sweetener, lingering drying feeling in the mouth and harsh mouthfeel) that would otherwise exist in the product. The taste-masking agent of the present invention is an extract of the *Angelica* sinensis root, which is a mixture of *Angelica* root components that are soluble in water and/or the organic solvents.

The terms "taste-masking effective amount" and "taste-improving amount" mean the same and refer to an amount at which a taste-masking agent, when used in a product including a food product, a dental product, an oral hygiene product or a medicinal product, results in improved palatability by reducing an undesirable taste that would otherwise exist in the product.

The term "taste-masking composition" means a composition that provides perceived reduction of an undesirable taste that would otherwise exist in a product such as a food product, a dental product, an oral hygiene product or a medicinal product. The taste-masking composition of the present invention comprises a taste-masking effective amount ranging from 1 to 5000 ppm by weight of the composition of Angelica sinensis root extract. The taste-masking composition of the present invention improves the palatability of a food product, a dental product, an oral hygiene product or a medicinal product by reducing an undesirable taste that would otherwise exist in these products.

The taste-masking effective amount of the *Angelica* sinensis root extract obtained from the disclosed process of the present invention, when employed in a product including a food product, a dental product, an oral hygiene product or a medicinal product, is 1 to 5000 ppm by weight and more preferably from about 10 to about 1000 ppm. The amount of extract used can vary and may be dependent upon the amount of ingredient, which imparts the off-tastes, used in the product; the purity of the extract or compounds thereof; the nature of the product; and/or whether the extract is a component of the product (*i.e.,* an additive) or topping. When used in baked goods and breakfast cereals including whole grain products, the *Angelica* root extract of the present invention can be employed at a taste-masking effective amount of from about 50 to about 5000 ppm, preferably from about 50 to about 1000 ppm and more preferably from about 100 to about 500 ppm. When used in meat and poultry products, soups, sauces such as gravies and dressings, pasta and pasta-like food products, the *Angelica* root extract of the present invention can be employed at a taste-masking effective amount of from about 10 to about 200 ppm and preferably from about 50 to about 100 ppm. When used in seasonings and snack food products, the *Angelica* root extract of the present invention can be employed at a taste-masking effective amount of from about 50 to about 200 ppm and preferably from about 100 to about 150 ppm. When used in chewing gums, confectionaries, hard and soft candies and dairy products such as yogurt, the *Angelica* root extract of the present invention can be employed at a taste-masking effective amount of from about 10 to about 100 ppm and preferably from about 50 to about 75 ppm. When used in beverages, the *Angelica* root extract of the present invention can be employed at a taste-masking effective amount of from about 5 to about 100 ppm and preferably from about 50 to about 75 ppm. Further, more taste-masking effective amount desirable for additional food products may be readily adjusted and determined by the skilled in the art in reference to the extraction process and taste-masking effective amount established by the present invention.

Baked goods and breakfast cereals such as whole grain products are typically prepared from flour obtained by milling, for example, wheat (*e.g*., durum, hard red spring, hard red winter, soft red spring, soft red winter, hard white, soft white, unclassed wheat, and mixed wheat), sorghum, milo, triticale, emmer, einkorn, spelt, oats, corn, rye, barley, rice, millet, buckwheat, quinoa, amaranth, African rice, popcorn, teff, canary seed, Job's tears, wild rice, tartar buckwheat, variants thereof, or mixtures thereof. Examples of whole grain products that would benefit from the use of the extract of the invention include, but are not limited to, a bagel, a biscuit, a bread, a bun (*e.g.,* hot dog or hamburger bun), a roll, a croissant, a dumpling, an English muffin, a muffin, a pita bread, a quickbread, a refrigerated/frozen dough products, dough, a tortilla, a pot pie, a ready to eat cereal, a ready to eat meal, stuffing, a microwaveable meal, a brownie, a cake, a coffee cake, a cookie, a dessert, a pastry, a sweet roll, a pie crust, baby food, a baking mix, a batter, a breading, a gravy mix, a meat substitute, a seasoning mix, a soup mix, a noodle, a pasta, ramen noodles, chow mein noodles, lo mein noodles, an ice cream cone, an ice cream sandwich, a cracker, a crouton, a doughnut, an egg roll, an extruded snack, a fruit and grain bar, a microwaveable snack product, a nutritional bar, a pancake, a par-baked bakery product, a pretzel, a granola-based product, a snack chip, a snack food, a snack mix, a waffle, a pizza crust, an instant beverage mix, a ready-to-drink beverage, a nutritional bar, a wafer, a chewable tablet, animal food or pet food. In some embodiments, a whole grain food product that includes an extract of the present invention may have an organoleptic profile substantially similar to a same food product made from the refined flour constituent in the absence of the extract or a substantially improved organoleptic profile compared to the same food product made in the absence of the extract. Other food products in which the extract of the invention can be used include, but are not limited to, alcoholic and non-alcoholic beverages (*e.g*., coffee; tea; wine; beverages containing wine; beer; beverages containing beer; liqueurs; schnapps; brandies; sodas containing fruit; isotonic beverages; soft drinks; nectars; fruit and vegetable juices and fruit or vegetable preparations; instant beverages, such as instant cocoa beverages, instant tea beverages and instant coffee beverages; ready to drink liquid drinks; liquid drink concentrates; and powdered drinks), broths or products containing broths (*e.g*., gravy sauces or mixes, instant soups, powdered soups, precooked soups, stews, and frozen entrees), as well as other food products that impart a bitter or astringent off-taste/linger. Other such foods include, but are not limited to snack foods (*e.g.,* potato, tortilla, vegetable or multigrain chips; pretzels or extruded snacks), confectionaries (*e.g*., chocolate, chocolate bar products, other products in bar form, fruit gums, hard and soft caramels and chewing gum), dairy Products (*e.g*., milk beverages, ice milk, yogurt, kefir, cream cheese, soft cheese, hard cheese, powdered milk, whey, butter, buttermilk and partially or fully hydrolyzed milk protein-containing products, flavored milk), soya protein or other soybean fractions (*e.g*., soya milk and products produced therefrom, soya lecithin-containing preparations, fermented products such as tofu or tempeh or products produced therefrom and soy sauces), and spice blends.

The invention is described in greater detail by the following non-limiting examples.

### Example 1: Preparation of Water Extract of Angelica Root

*Angelica sinensis* root (100 g, dried, cut, and sifted) was placed in a jacketed percolator. The root sample was extracted with 500 mL water by continuous percolation at 70 °C for 3 hours. The water extract was then discharged and collected. The extraction procedure was repeated twice, each time with 300 mL water, under the same percolation conditions. The water extracts were then pooled, clarified by filtration and dried to provide a powder of *Angelica* root extract. The yield was approximately 40 to 45 g.

### Example 2: Preparation of Washed Water Extract of Angelica Root

*Angelica sinensis* root (100 g, dried, cut, and sifted) was placed in a jacketed percolator. The root sample was extracted with 500 mL water by continuous percolation at 70 °C for 3 hours. The water extract was then discharged and collected. The extraction procedure was repeated twice, each time with 300 mL water, under the same percolation conditions. The water extracts were then pooled and concentrated to a volume of approximately 300 mL using a conventional drying method. The concentrated extract was then washed (liquid-liquid extraction) with 100 mL 2-butanol. The 2-butanol layer was discarded and the aqueous layer was washed two more times with the same amount of 2-butanol. The washed aqueous layer was then dried to provide a powder of *Angelica* root extract. The yield was approximately 40 to 45 g.

### Example 3: Preparation of Ethanol Extract of Angelica Root

*Angelica sinensis* root (100 g, dried, cut, and sifted) was placed in a jacketed percolator. The root sample was extracted with 500 mL ethanol (190 proof) by continuous percolation at 70 °C for 3 hours. The ethanolic extract was then discharged and collected. The extraction procedure was repeated twice, each time with 300 mL ethanol (190 proof), under the same percolation conditions. The ethanolic extracts were then pooled, clarified by filtration and dried to provide a powder of *Angelica* root extract. The yield was approximately 20 to 30 g.

### Example 4: Preparation of Ethanol-Water (70/30 v/v) Extract of Angelica Root

*Angelica sinensis* root (100 g, dried, cut, and sifted) was placed in a jacketed percolator. The root sample was extracted with 500 mL of a solvent mix composed of ethanol (190 proof) and water at a volume ratio of 70:30, by continuous percolation at 70 °C for 3 hours. The ethanol-water extract was then discharged and collected. The extraction procedure was repeated twice, each time with 300 mL of the solvent mix, under the same percolation conditions. The ethanol-water extracts were then pooled, clarified by filtration and dried to provide a powder of *Angelica* root extract. The yield was approximately 40 to 50 g.

### Example 5: Pasta Containing Angelica Root Water Extract

A dry powdered sample of *A. sinensis* root extract was prepared according to Example 2. Dried root of *A. sinensis* was extracted with hot water, and the water extract was washed (liquid-liquid extraction) with 2-butanol to remove intrinsic flavors of *Angelica.* The washed extract was then dried into a powdered form. The dry powdered sample of *A. sinensis* root extract was applied at 10, 25 or 100 ppm in a formulated mix of whole wheat flour (42.86 g), semolina flour (25.72 g), egg white powder (2.86 g), and water (28.56 g) that was then processed into pasta. The presence of the *A. sinensis* root extract masked the bitter and astringent off-taste of the whole wheat pasta.

As with *A. sinensis,* a water extract from *A. archangelica* root also improved the taste of whole grain pasta. Therefore, the water extract of this invention can be obtained from any species of *Angelica,* in particular those species known to be edible.

### Example 6: Wheat Crackers Containing Angelica Root Water Extract

A dry powdered sample of *A. sinensis* root extract, as prepared in Example 2, was applied at 100 ppm in a wheat cracker product (Table 1).

**TABLE 1**

| Ingredient | Grams | % |
|---|---|---|
| Whole Wheat Flour | 158.25 | 63.30 |
| Sugar | 4.85 | 1.94 |
| Salt | 2.38 | 0.95 |
| Paprika | 0.60 | 0.24 |
| Butter | 23.75 | 9.50 |
| Water | 59.40 | 23.76 |
| Vanilla | 0.78 | 0.31 |
| Total | 250 | 100.00 |

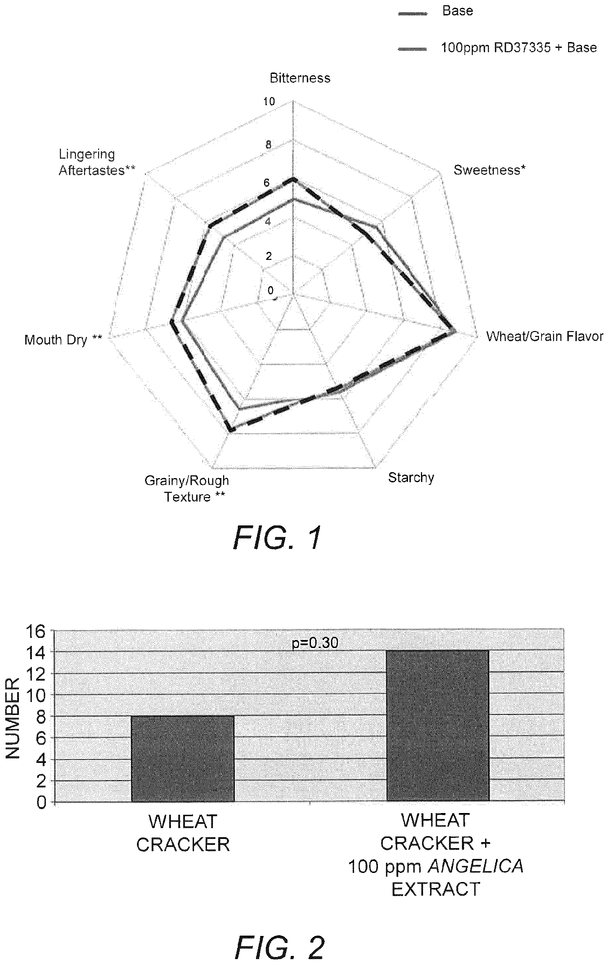

The treated sample had significantly less bitterness, grainy-rough texture, mouth dryness, and lingering aftertaste than the control sample (Table 2, Figure 1). Further, while the cracker with the *A. sinensis* root extract was directionally sweeter than base, there was no significant different between the two samples in wheat/grain and starchy flavors (Table 2).

**TABLE 2**

| Flavor Profile | Base | Extract + Base |
|---|---|---|
| Bitterness** | 6.0 | 5.0 |
| Sweetness* | 5.0 | 5.6 |
| Wheat/Grain Flavor | 8.7 | 8.8 |
| Starchy | 5.4 | 5.5 |
| Grainy/Rough Texture** | 7.7 | 6.6 |
| Mouth Dry** | 6.7 | 6.1 |
| Lingering Aftertastes** | 5.6 | 4.7 |

Numbers represent means, n=11. **There was a significant difference between the two samples at p=0.05. * Directional difference at p=0.10.

Furthermore, as illustrated in Figure 2, 14 of the 22 participating panelists preferred the treated cracker in a paired comparison test.

### Example 7: Whole Wheat Pan Bread Containing Angelica Root Water Extract

A whole wheat (100%) pan bread mixture is prepared using the ingredients listed in Table 3, wherein *Angelica* root extract prepared according to Example 1, is added to the water. All ingredients are placed in a mixer and mixed on low speed for one minute. Subsequently, the mixer speed is increased and the dough is mixed for an addition 8 to 12 minutes. A dough temperature of 80 to 82 °F is maintained and the dough is rested for 5 minutes. The dough is divided into 130 gram pieces and rested an additional 10 minutes. The dough pieces are proofed for 60 to 90 minutes at 105 °F and subsequently baked for 12.5 minutes at 380 °F.

**TABLE 3**

| Ingredient | Grams | % |
|---|---|---|
| Whole Wheat Flour | 225.00 | 48.24 |
| Water | 171.00 | 36.66 |
| Gluten | 18.00 | 3.86 |
| Instant Dry Yeast | 3.15 | 0.68 |
| Soybean Oil | 6.75 | 1.45 |
| Yeast Food (non-bromate) | 0.86 | 0.18 |
| Sodium Stearoyl Lactylate (EMPLEX) | 1.13 | 0.24 |
| Granulated Sugar | 18.00 | 3.86 |
| Honey | 4.50 | 0.96 |
| Molasses | 11.25 | 2.41 |
| Salt | 4.50 | 0.96 |
| Glycerol Monostearate (GMS-90) | 2.25 | 0.48 |
| Ascorbic Acid | 0.04 | 0.01 |
| Azodicarbonamide (ADA) | 0.01 | 0.00 |
| Total | 466.44 | 100.00 |

### Example 8: Whole Wheat Roll Containing Angelica Root Water Extract

A whole wheat (51%) roll mixture is prepared as follows. The ingredients listed in Table 4 are combined for brew and incubated until frothy (at least 15 minutes).

**TABLE 4**

| Ingredient | Grams | Formula % | Total Dough % |
|---|---|---|---|
| Instant Dry Yeast | 12.50 | 21.55 | 3.00 |
| High Fructose Corn Syrup 55 (HFCS) | 13.63 | 23.50 | 3.27 |
| Water (90 °F) | 28.95 | 49.92 | 6.95 |
| Total | 58 | 94.97 | |

White flour containing powdered *Angelica* root extract, prepared according to Example 1, is combined with ingredients for Stage 1 (Table 5) and mixed with a paddle at a low speed. Subsequently, ingredients from Stage 2 (Table 5) are added and the mixture is mixed on low speed to form dough (about 30 seconds). Using a dough hook, the mixture is further mixed an additional 9 minutes until the gluten has developed. A dough temperature of approximately 83 °F is maintained and the dough is allowed to rise in a proof box until doubled (about 1 hour). The dough is punched down and cut into 76.5 gram pieces. Each piece is formed to a four inch hamburger bun pan and allowed to rise in a proof box until the buns crest the top (about 45 minutes). The buns are subsequently baked in a preheated convection oven at 350 °F, with 3 seconds of steam, for approximately 9 minutes, wherein the steam is vented 4 minutes into the baking period.

**TABLE 5**

| Ingredient | Grams | Formula % | Total Dough % |
|---|---|---|---|
| *Stage 1* | | | |
| Flour with Extract | 204.18 | 24.61 | 49.00 |
| Whole Wheat Flour | 212.52 | 25.61 | 51.00 |
| Salt | 8.33 | 1.00 | 2.00 |
| Vital Wheat Gluten | 20.83 | 2.51 | 5.00 |
| Calcium Propionate | 1.25 | 0.15 | 0.30 |
| PANODAN 140K* | 1.45 | 0.18 | 0.35 |
| Ascorbic Acid | 0.140 | | 0.034 |
| | | | |

| *Stage 2* | | | |
|---|---|---|---|
| Water (70°F) | 235 | 28.32 | 56.39 |
| HFCS | 75.5 | 9.10 | 18.12 |
| Soybean Oil | 12.5 | 1.51 | 3.00 |
| Brew (Table 2) | 58 | 6.99 | 13.92 |
| Total | 829.7 | 100 | |

| | | | |
|---|---|---|---|
| *Blend of diacetyl tartaric acid ester of mono-diglycerides (DATEM), wheat starch and alpha amylase. | | | |

### Example 9: Wheat Crackers Containing Angelica Root Ethanol Extract

A whole wheat cracker product is prepared according to Example 6, wherein a dry powdered sample of *A. sinensis* root extract prepared according to Example 3, is applied at 100 ppm. The presence of the *A. sinensis* root extract masked the bitter and astringent off-taste of the wheat crackers.

### Example 10: Wheat Crackers Containing Angelica Root Ethanol-Water Extract

A whole wheat cracker product is prepared according to Example 6, wherein a dry powdered sample of *A. sinensis* root extract prepared according to Example 4, is applied at 100 ppm. The presence of the *A. sinensis* root extract masked the bitter and astringent off-taste of the wheat crackers.

### Example 11: Other Masking Properties of Angelica Root Extract

In addition to whole grain food, it was determined whether the *Angelica* root extract of the present invention prepared according to Example 1 could mask undesirable properties of other products.

*Orange Flavored Still Beverage* + *Rebaudioside A.* An orange-flavored still beverage was prepared, which contained 0.05% flavor, 0.01% citric acid and 60 ppm rebaudioside A (Reb A). To this was added 20 ppm of *Angelica* root extract. The control beverage without *Angelica* root extract imparted an intense Reb A, metallic, off-taste/linger, as well as a bitter finish and artificial sweetener-like off-taste. In comparison, the beverage containing the *Angelica* root extract imparted less Reb A off-taste/linger and less bitter compared to the control. Moreover, there was a reduction in the artificial sweetener-like off taste.

*Orange Flavored Still Beverage* + *Luo Han.* An orange-flavored still beverage was prepared, which contained 0.05% flavor, 0.01% citric acid and 100 ppm Luo Han. To this was added 20 ppm of *Angelica* root extract. The control beverage without *Angelica* root extract imparted a long-lasting artificial sweetener-like linger in the throat. In comparison, the beverage containing the *Angelica* root extract imparted less off-sweet linger compared to the control. Moreover, the *Angelica* root extract-containing beverage cleaned well after swallowing.

*Orange Flavored Still Beverage* + *Sucralose.* An orange-flavored still beverage was prepared, which contained 0.05% flavor, 0.01% citric acid and 100 ppm Sucralose. To this was added 20 ppm of *Angelica* root extract. The control beverage without *Angelica* root extract imparted a typical artificial sweetness, bitterness and linger. In comparison, the beverage containing the *Angelica* root extract had a reduction in bitterness and sweetness linger compared to the control.

*Unsalted Chicken Broth.* To an unsalted (0.2% KCl) chicken broth was added 50 ppm of *Angelica* root extract. The control broth without *Angelica* root extract imparted a metallic, mineral, and bitter off-taste/linger. In comparison, the broth containing the *Angelica* root extract had a reduction in metallic, mineral, and bitter off-taste/linger compared to the control. In addition, the test broth had a more pleasant salt-like taste.

*Water* + *Menthol (10 ppm).* To a water/menthol solution was added 20 ppm of *Angelica* root extract. The control solution without *Angelica* root extract imparted bitter, astringent, mouth drying, and "menthol burn" off-tastes. In comparison, the *Angelica* root extract suppressed the bitter, astringent, mouth drying, and "menthol burn" off-tastes.

### Example 12: Taste-Masking Effective Amount of Angelica Root Extract

Tasting-masking effective amount of the *Angelica* root extract of the present invention prepared according to Example 1 were further investigated using the following samples.

*Water* + *Stevia Extract (Steviol Glycosides-Containing) (800 ppm).* To three identical samples of Stevia extract solution were added *Angelica* root extract of concentrations of 10, 50 and 100 ppm, respectively. At 10 and 50 ppm, the *Angelica* root extract suppressed the metallic and bitter off-taste as well as the artificial sweetener-like linger. At 100 ppm, the taste-masking effect of the *Angelica* root extract reached plateau and a toasted grainy off-taste was noted.

*Water* + *Menthol (30 ppm).* To three identical samples of menthol solution were added *Angelica* root extract of concentrations of 10, 50 and 100 ppm, respectively. At 10 ppm, the *Angelica* root extract suppressed the bitter, astringent, mouth drying and the "menthol burn" off-tastes. At 50 ppm, the taste-masking effect showed well but a hint of toasted grainy off-taste was noted. At 100 ppm, the taste-masking effect overpowered the cool and refreshing menthol flavors and the extract itself imparted herbal off-taste.

*Water* + *KCl (0.2%).* To three identical samples of KCl solution were added *Angelica* root extract of concentrations of 10, 50 and 100 ppm, respectively. At 10 ppm, the *Angelica* root extract suppressed the metallic, mineral and bitter off-tastes as well as the lingering feeling. A hint of herbal off-taste was noted. At 50 ppm, the taste-masking effect showed well but the grainy and herbal off-tastes developed. At 100 ppm, the taste-masking effect showed well. The grainy, toasted and herbal off-tastes became strong.

*Water* + *Soy Isolate Powder (GNC)* (8%). To three identical samples of soy solution were added *Angelica* root extract of concentrations of 100, 500 and 1000 ppm, respectively. At 100 ppm, the *Angelica* root extract barely suppressed the bitter, grainy, beany, green and grassy off-tastes. There was no off-taste caused by the extract itself. At 500 ppm, the taste-masking effect showed well without off-taste. At 1000 ppm, the herbal off-taste from the extract was strong.

*Wheat Cracker.* A sample of wheat cracker without the *Angelica* root was prepared according to Example 6. To five identical samples of wheat crackers were added *Angelica* root extract of concentrations of 100, 500, 1000, 5000 and 10,000 ppm, respectively. At 100 ppm, the *Angelica* root extract suppressed bitterness, grainy-rough texture, mouth dryness, and lingering aftertaste. At 500 and 1000 ppm, the taste-masking effect showed well. At 5000 ppm, the taste-masking effect showed well but slight herbal and celery off-tastes were noted. At 10,000 ppm, the taste-masking effect overpowered and the off-taste of the extract such as herbal and celery notes became strong.

## Claims

1. A method of reducing an undesirable taste in a product selected from the group consisting of a food product, a dental product, an oral hygiene product and a medicinal product comprising the step of incorporating a taste-masking composition,
wherein the taste-masking composition comprises a taste-masking effective amount ranging from 1 to 5000 ppm by weight of the composition of an *Angelica* root extract, and
wherein the *Angelica* root extract is prepared by a process comprising the steps of:
(i) submerging a root of an *Angelica sinensis* in a solvent selected from the group consisting of water, an organic solvent and a mix thereof;
(ii) circulating the solvent continuously through the root of the *Angelica sinensis* at a temperature ranging from room temperature to 100 °C to provide a soluble extract; and
(iii) collecting the soluble extract from step (ii) and evaporating the solvent to provide the *Angelica root* extract.

2. The method of claim 1, wherein the product is the food product, and wherein the food product is selected from the group consisting of a beverage, a broth and a whole grain food product.

3. The method of claims 1 or 2, wherein the taste-masking effective amount is from 10 to 1000 ppm by weight of the composition.

4. The method of any preceding claim, wherein the organic solvent is an alcohol; and optionally or preferably wherein the alcohol is an ethanol.

5. The method of any preceding claim, wherein the temperature ranges from 70 to 80 °C.

## Patentansprüche

1. Verfahren zum Verringern eines unerwünschten Geschmacks in einem Produkt ausgewählt aus der Gruppe bestehend aus einem Lebensmittelprodukt, einem Dentalprodukt, einem Mundhygieneprodukt und einem medizinischen Produkt, umfassend den Schritt des Einverleibens einer geschmacksmaskierenden Zusammensetzung,
wobei die geschmacksmaskierende Zusammensetzung eine geschmacksmaskierend wirksame Menge in dem Bereich von 1 bis 5000 ppm, bezogen auf das Gewicht der Zusammensetzung, eines Angelikawurzelxtrakts umfasst, und
wobei der Angelikawurzelextrakt durch ein Verfahren hergestellt ist, das die Schritte umfasst:
(i) Eintauchen einer Wurzel einer *Angelica sinensis* in ein Lösungsmittel ausgewählt aus der Gruppe bestehend aus Wasser, einem organischen Lösungsmittel und einem Gemisch davon;
(ii) kontinuierliches Zirkulieren des Lösungsmittels durch die Wurzel der *Angelica sinensis* bei einer Temperatur in dem Bereich von Raumtemperatur bis 100 °C, um einen löslichen Extrakt zu ergeben; und
(iii) Sammeln des löslichen Extrakts aus Schritt (ii) und Abdampfen des Lösungsmittels, um den Angelikawurzelextrakt zu ergeben.

2. Verfahren gemäß Anspruch 1, wobei das Produkt das Lebensmittelprodukt ist und wobei das Lebensmittelprodukt ausgewählt ist aus der Gruppe bestehend aus einem Getränk, einer Brühe und einem Vollkorn-Lebensmittelprodukt.

3. Verfahren gemäß Ansprüchen 1 oder 2, wobei die geschmacksmaskierend wirksame Menge von 10 bis 1000 ppm, bezogen auf das Gewicht der Zusammensetzung, ist.

4. Verfahren gemäß einem der vorstehenden Ansprüche, wobei das organische Lösungsmittel ein Alkohol ist; und gegebenenfalls oder vorzugsweise wobei der Alkohol Ethanol ist.

5. Verfahren gemäß einem der vorstehenden Ansprüche, wobei die Temperatur in dem Bereich von 70 bis 80 °C liegt.

## Revendications

1. Méthode de réduction d'un goût indésirable dans un produit choisi dans le groupe constitué par un produit alimentaire, un produit dentaire, un produit d'hygiène orale et un produit médicinal, comprenant l'étape consistant à incorporer une composition de masquage de goût,
dans laquelle la composition de masquage de goût comprend une quantité efficace de masquage de goût allant de 1 à 5000 ppm en poids de la composition d'un extrait de racine d*'Angelica,* et
où l'extrait de racine d*'Angelica* est préparé par un procédé comprenant les étapes consistant à :
(i) submerger une racine d*'Angelica sinensis* dans un solvant choisi dans le groupe constitué par l'eau, un solvant organique et un mélange de ceux-ci ;
(ii) faire circuler le solvant de manière continue dans la racine d*'Angelica sinensis* à une température allant de la température ambiante à 100°C, afin de fournir un extrait soluble ; et
(iii) récupérer l'extrait soluble issu de l'étape (ii) et évaporer le solvant afin de fournir l'extrait de racine d'*Angelica.*

2. Méthode selon la revendication 1, dans laquelle le produit est un produit alimentaire, et où le produit alimentaire est choisi dans le groupe constitué par une boisson, un bouillon et un produit alimentaire à base de grains entiers.

3. Méthode selon les revendications 1 ou 2, dans laquelle la quantité efficace de masquage de goût va de 10 à 1000 ppm en poids de la composition.

4. Méthode selon l'une quelconque des revendications précédentes, dans laquelle le solvant organique est un alcool ; et éventuellement ou préférablement où l'alcool est un éthanol.

5. Méthode selon l'une quelconque des revendications précédentes, dans laquelle la température va de 70 à 80°C.
